# EUROPEAN PATENT APPLICATION

(11) **EP 4 279 010 A1**
(43) Date of publication of application: **22.11.2023**
(21) Application number: 22305754.8
(22) Date of filing: 20.05.2022
(51) Int. Cl.: A61B 34/30, A61B 34/20

(54) **COMPUTER-ASSISTED SURGERY SYSTEM WITH PASSIVE TOOL-HOLDER**

(71) Applicant: KAT ROBOTICS, 38400 Saint-Martin-d'Hères (FR)
(72) Inventor: LAVALLEE, Stéphane, 38400 SAINT MARTIN D'HERES (FR); DEHAN, Christophe, 38400 SAINT MARTIN D'HERES (FR)
(74) Representative: Regimbeau

(57) **Abstract**

The present invention concerns a computer-assisted surgery system (100) for treating a planar region of interest of an anatomical structure with a surgical tool (130), the computer-assisted surgery system comprising:
• a robotic arm with at least three motorized joints,
• a passive tool holder (120) attached to a flange (112) of the robotic arm,
• the surgical tool (130) attached to the passive tool holder (120), the surgical tool (130) being adapted to be displaced, manually, within a working range (R),
• a localization unit adapted to determine, dynamically, a relative pose of the working range (R) with respect to the region of interest,
• a control unit adapted to compute and send at least one first instruction to at least one of the motorized joints in order for the working range (R) to overlap, at least partially, the region of interest,
the control unit being configured to:
• compare the relative position of the active portion (133) of the surgical tool (130) with respect to the working range (R) with a targeted position (153) of the active portion (133) of the surgical tool (130) within the working range (R),
• determine a pose of an updated working range (R') so as for the active portion (133) of the surgical tool (130) to be located at the targeted position (153) within said updated working range (R'),
• determine an updated pose of the flange (112) so as for the working range (R) to be displaced to the updated working range (R').

## Description

The present invention pertains to the domain of computer-assisted surgery systems. Especially, the invention concerns a computer-assisted surgery system which comprises a passive part adapted to be used for performing orthopedics surgeries.

The state of the art prior to robotic assistance, as the use of bone attached templates and guides to guide hand-held saw or other tools, limits resulting accuracy to surgeons' dexterity, in particular due to inaccuracy in guide placement and stability over patient anatomy and mandatory free mechanical play between tool and guide. Guide placement is intrusive as it also requires large access opening and fixation over bony structure. Using a robot to assist in cutting a plane in a bone with a cutting instrument such as a surgical saw, is now a well-known solution in orthopedic surgery. Different solutions have been proposed over the last few decades aimed to provide better accuracy, lower invasiveness, and lower complication rates, all contributing to expected better patient outcomes.

In a first approach, such as the one developed in the robot Rosa^{®} of the company Zimmer-Biomet, the robot carries a cutting guide, aligned with the plane to be cut. The surgeon thus has to insert the cutting instrument through the cutting guide to perform the planned cut. The robot here is adapted to maintain the cutting guide aligned with the plane to be cut. At least one drawback of such system is that it adds inaccuracy as it does not permit to take into account the play and bending of the cutting instrument in the cutting guide.

In a second approach, such as the robot Velys^{®} developed by Depuy Synthes, the robot carries a planar mechanism to which the cutting instrument is attached. The robot is here used to maintain the cutting instrument within the predefined plane to ensure that the surgeon who operates the cutting instrument follows the planned cut. In this second approach, the deviations of the saw from the planned plane are compensated thanks to an optical localization unit encompassing at least one tracker attached to the cutting instrument and at least a second tracker attached to the bone to be cut. At least one drawback of this kind of system is that the tracker attached to the cutting instrument must remain visible at any time for a camera of the localization unit which can be challenging considering the multiple angled positions that the cutting instrument must be put on during the cut(s). The presence of surgeon and helping hands around the surgery zone also typically results in frequent loss of line of sight between optical localization camera and tracker. The tracker also hampers the view of the surgery zone for the surgery personnel. Additionally, as the cutting instrument is, by definition, brought close to the anatomical structure treated, it can be soiled by blood or tissue projections. As well known in the art, a soiled tracker can result in a wrong evaluation of the pose of the associated object which adds inaccuracy or can be dangerous during such a surgical procedure.

Thus, it remains a need for an improved system which can determine and/or maintain, in real-time, the precise pose of the cutting tool with respect to the planned cutting plane.

The present invention meets this objective whilst letting the surgeon freely move the saw or tool to remove bony material within that plane, combining robot's high accuracy and surgeon's expertise. Some optical localization line of sight issues, tracker close exposure to biologic projections and surgeon's cleared limited view of the surgery zone are also improved by the invention.

An object of the present invention thus concerns a computer-assisted surgery system for treating a planar region of interest of an anatomical structure with a surgical tool, the computer-assisted surgery system comprising:
- a robotic arm with at least three motorized joints,
- a passive tool holder attached to a flange of the robotic arm,
- the surgical tool attached to the passive tool holder, the surgical tool being adapted to be displaced, manually, within a working range, the working range being formed as a planar area, a geometry of this working range being determined by a geometry of the passive tool holder and a pose of this working range being determined by a pose of the flange,
- a storage unit storing, at least, a geometry of the surgical tool and a targeted position of an active portion of the surgical tool within the working range,
- a localization unit adapted to determine, dynamically, a relative pose of the working range with respect to the region of interest,
- a control unit adapted to receive from the localization unit the relative pose of the working range with respect to the region of interest and to dynamically compute and send at least one first instruction to at least one of the motorized joints in order for the working range to overlap, at least partially, the region of interest,
the control unit being configured to:
- receive from the localization unit, dynamically, a relative position of the surgical tool with respect to the working range,
- determine a relative position of an active portion of the surgical tool with respect to the working range based on the received relative position of the surgical tool with respect to the working range and on the stored geometry of the surgical tool,
- compare the determined relative position of the active portion of the surgical tool with respect to the working range with the stored targeted position of the active portion of the surgical tool within the working range,
- determine a pose of an updated working range so as for the active portion of the surgical tool to be located at the targeted position within said updated working range, if the received relative position of the active portion of the surgical tool with respect to the working range is different from the targeted position,
- determine an updated pose of the flange so as for the working range to match the determined pose of the updated working range.

The invention may be complemented by one or several of the following features, alone or in combination.

The region of interest is defined by a contour, a first part of this contour forming a protective boundary and a second part of this contour forming an entry zone to the region of interest, and the control unit is further configured to
- determine if the updated working range will remain within the protective boundary of the region of interest,
- compute at least one second instruction(s) adapted to be sent to at least one of the motorized joints of the robotic arm in order to displace the flange of the robotic arm to its updated pose, if it is determined that the updated working range will remain within the protective boundary of the region of interest,
- execute the at least one second instruction(s).

In the present specification, the words "send the instruction(s)" and "execute the instruction(s)" are used without distinction.

The region of interest is defined by a contour, a first part of this contour forming a protective boundary and a second part of this contour forming an entry zone to the region of interest, and the control unit is further configured to
- determine if the updated working range will extend beyond the protective boundary of the region of interest,
- modify the updated pose of the working range in order for a border of the updated working range to match the protective boundary of the region of interest, if it is determined that the updated working range will extend beyond the protective boundary of the region of interest,
- modify the updated pose of the flange so as for the working range to be displaced to the modified updated working range.
- compute at least one second instruction(s) adapted to be sent to at least one of the motorized joints of the robotic arm in order to displace the flange of the robotic arm to its modified updated pose,
- execute the at least one second instruction(s).

According to an aspect of the invention, the working range comprises a central zone surrounded by a peripheral zone, and the control unit can be configured to
- execute the second instruction(s) as soon as the surgical tool enters the peripheral zone.

The peripheral zone can represent up to 30% of the working range.

According to another aspect of the invention, determining the pose of the updated working range comprises:
- determining at least one parameter related to the displacement of the surgical tool based on the received relative position of the surgical tool with respect to the working range;
- selecting the displacements to be considered for determining the pose of the updated working range, based on the at least one parameter.

The at least one parameter is one or several of the following:
- a displacement acceleration of the surgical tool
- a displacement speed of the surgical tool
- a displacement amplitude of the surgical tool
- a frequency of the displacement of the surgical tool.

Selecting the displacements to be considered for determining the pose of the updated working range can comprise:
- determining the at least one parameter associated with the displacement of the surgical tool,
- comparing the determined parameter(s) with a predefined range
- ignoring the displacements having parameter(s) outside the predefined range while determining the pose of the updated working range.

The localization unit comprises:
- a first subsystem adapted to determine, dynamically, the relative pose of the surgical tool with respect to the working range,
- a second subsystem adapted to determine, dynamically, the relative pose of the flange of the robotic arm with respect to the region of interest,
the first subsystem comprising at least one positional sensor adapted to determine, dynamically, a geometry of the passive tool-holder, and the second subsystem comprising an optical tracking unit and/or an electromagnetic tracking unit and/or an inertial tracking unit adapted to determine, dynamically, the relative pose of the flange with respect to the anatomical structure.

According to a first embodiment of the invention, the passive tool holder comprises at least a first segment and a second segment linked to one another thanks to a first passive joint rotatable around a first axis, the first segment being attached to the flange of the robotic arm thanks to a second passive joint rotatable around a second axis parallel to the first axis, and the second segment being attached to an attaching part of the surgical tool thanks to a third passive joint rotatable around a third axis parallel to the first axis and to the second axis.

According to the first embodiment of the invention, each passive joint can be equipped with at least one angular sensor adapted to determine, dynamically, an angular position of the concerned passive joint, the at least one angular sensor being part of the first subsystem of the localization unit.

According to a second and a third embodiments of the invention, the passive tool holder comprises at least one opened housing which cooperates with an attaching part of the surgical tool, the housing comprising a guiding device adapted to guide and limit the displacement of the surgical tool within the working range and the attaching part of the surgical tool allowing rotation of the surgical tool around an axis perpendicular to the working range.

According to the second embodiment, the passive tool holder comprises a first opened housing and a second opened housing, the guiding device comprising at least a first rail mounted within the first opened housing and a second rail mounted within the second opened housing.

According to the third embodiment, the guiding device is formed as a blind hole formed within the housing.

The computer-assisted surgery system of the invention can comprise at least one force or torque sensor arranged between the flange of the robotic arm and the attaching part of the surgical tool. Advantageously, this force or torque sensor is arranged between the flange and the passive tool holder.

Further details and features of the invention are described below with reference to the following drawings:
- Figure 1 is a general perspective view of a computer-assisted surgery system according to a first embodiment of the present invention ;
- Figure 2 is a focused view of a passive tool holder of the computer-assisted surgery system according to the first embodiment of the present invention, represented during a surgery;
- Figure 3 is a diagram view of steps implemented by a control unit of the computer-assisted surgery system of the invention;
- Figure 4 is a representation of the movements of the computer-assisted surgery system according to a particular configuration of the invention;
- Figure 5a and 5b are focused views of the passive tool holder of the computer-assisted surgery system according to two variants of the first embodiment of the invention;
- Figure 6 is a focused view of the passive tool holder of the computer-assisted surgery system according to a second embodiment of the present invention;
- Figure 7 is a focused view of the passive tool holder of the computer-assisted surgery system according to a third embodiment of the present invention;
- Figure 8 is a partial top view of the passive tool holder according to the third embodiment of the present invention.

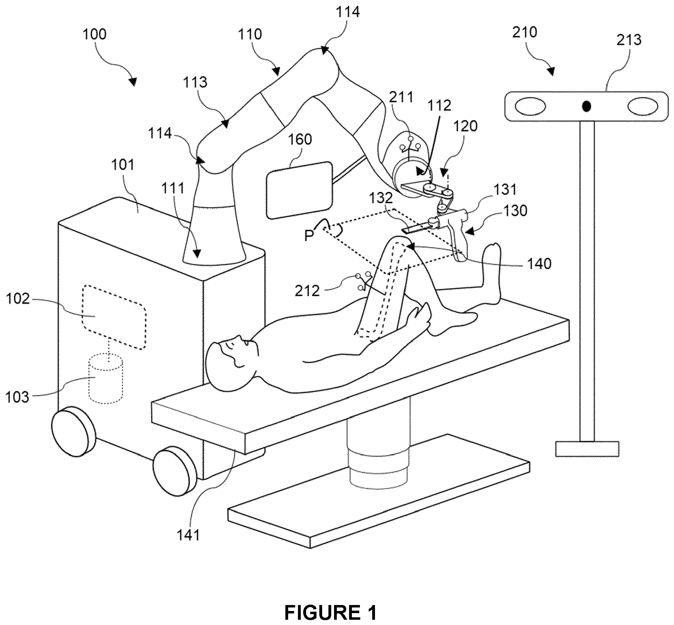

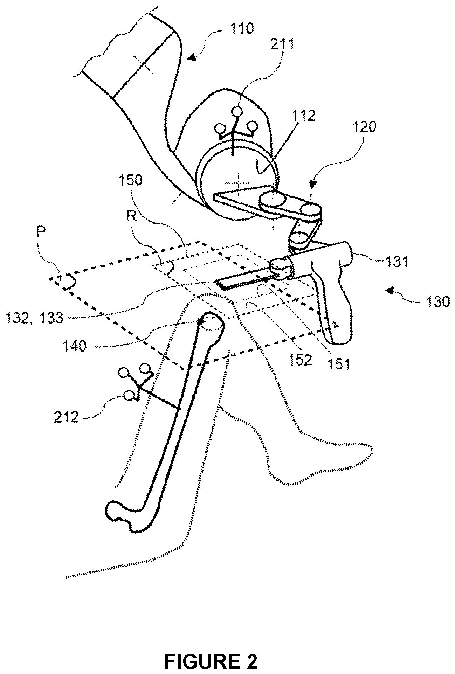

Figure 1 is a perspective view of a computer-assisted surgery system 100 according to the invention. The computer-assisted surgery system 100 comprises a base 101 from which extends a robotic arm 110 connected to a passive tool holder 120. For instance, the base 101 can be a wheeled cart. As shown, a surgical tool 130 is attached to the passive tool holder 120. The surgical tool 130 is adapted to treat a planar region of interest 140 of an anatomical structure. This planar region of interest 140 can be defined as an intersection between a planned surgical plane P and the concerned anatomical structure. According to the illustrated embodiment, the surgical tool 130 comprises at least one power tool 131 adapted to power a cutting tool 132. By "cutting tool" we here mean that the tool is adapted to cut bony structures. For instance, this cutting tool can be a saw, a burr, a drill, a laser, a water jet, a scalpel, focused ultrasounds or a shaver. As for instance referenced on figure 2, the cutting tool 132 comprises an active portion 133 which forms the part of the cutting tool especially adapted to perform the cuts. Advantageously, the surgical tool can be removably attached to the passive tool holder 120, so that it can be easily detached to be changed to another surgical tool or to be sterilized after a surgery for instance.

The robotic arm 110 extends between a first end 111 connected to the base 101 and a second end 112 which forms a flange of the robotic arm 110 to which the passive tool holder 120 is connected. The robotic arm 110 comprises several links 113 connected to one another thanks to motorized joints 114. According to the invention, the robotic arm 110 comprises at least three motorized joints 114 and it could comprise more than three motorized joints, for instance six motorized joints within the scope of the invention. Obviously, as illustrated on figure 1, the robotic arm could also comprise more than six motorized joints without departing from the scope of the invention.

Thanks to the passive tool holder 120, the surgical tool 130 can be freely moved within a working range R, for instance illustrated on figure 2. As shown, the working range R is formed as a planar area defined by a border 150. A geometry of the working range R is determined by a geometry of the passive tool holder 120 and a pose of this working range R is determined by a pose of the flange 112 of the robotic arm 110. To say it with other words, the relative pose of the flange 112 with respect to the working range R is fixed over time. Thus, for each displacement of the flange 112, the pose of the working range is modified. As illustrated on figure 2, the working range R also comprises a central zone 151 surrounded, at least partially, by a peripheral zone 152. According to an aspect of the invention, the peripheral zone represents up to 30% of the working range R.

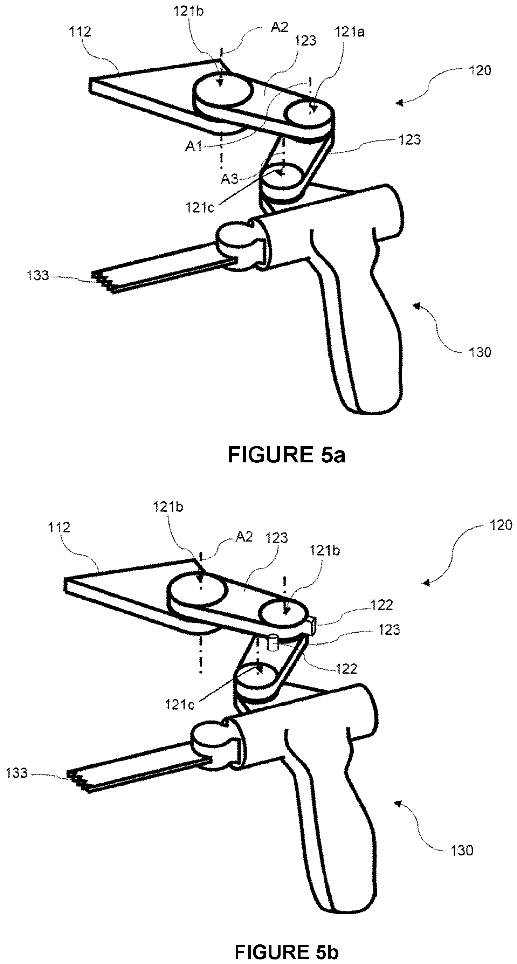

As detailed below with reference to figures 5a to 7, the passive tool holder 120 is designed to provide a surgical tool 130 which can be displaced according to three degrees of freedom, upon which two translations and one rotation.

As understandable from figures 1 and 2, the system of the invention is particularly adapted to perform horizontal cuts, i.e. cuts along planes parallel, or substantially parallel, to the ground. Nevertheless, some surgeries imply performing cuts along oblique or vertical planes, i.e. cuts along planes transverse or perpendicular to the ground. As the passive tool holder is passive, performing vertical cuts with it can be challenging. Indeed, it is understood that in a vertical or oblique position, the gravity would attract the surgical tool which would then drag the passive tool holder down. To prevent such a situation, the passive tool holder of the present invention can be locked in a retracted position, while the treatment of the region of interest is performed. In the locked retracted position, the passive tool holder cannot be displaced by the user, but only by movements of the robotic arm, thus making it insensitive to the gravity effect.

Referring back to figure 1, the computer-assisted surgery system 100 also comprises a control unit 102 and at least one storage unit 103, the control unit 102 being adapted to communicate with the storage unit 103. This communication can be wired or wireless without departing from the scope of the invention. According to the illustrated embodiment, the control unit 102 and the storage unit 103 are encompassed within the base 101 of the system, but they could be positioned anywhere else within the system, or even be deported within the scope of the invention. The storage unit 103 could also be part of the control unit 102 within the scope of the invention. The storage unit 103 is, for instance, used to store, at least, one or several of the following:
- geometries of the robotic arm, of the passive tool holder and/or of the surgical tool
- a relative pose of the region of interest with respect to the anatomical structure,
- the relative pose of the working range with respect to the flange
- a targeted position of the active portion of the surgical tool with respect to the working range.

In the present document, the geometries of the robotic arm, the passive tool holder and the surgical tool also comprise the geometries of the connections formed between these three objects.

The relative pose of the region of interest 140 with respect to the anatomical structure is determined during a planning procedure. During this planning procedure, a contour of the region of interest is defined. This contour comprises at least a first part which forms a protective boundary, that is to say a boundary set to protect tissues surrounding the region of interest, and at least a second part which forms an entry zone to the region of interest. Unlike the protective boundary, the second part forming the entry zone to the region of interest is adapted to be crossed by the surgical tool. Several well-known methods can be used to realize such planning, including imaging or not, and they are not developed in this document.

As shown, the computer-assisted surgery system 100 further comprises a localization unit which comprises at least a first subsystem adapted to determine, dynamically, relative poses of the surgical tool 130 with respect to the working range R, and a second subsystem 210 adapted to determine, dynamically, relative poses of the flange 112 of the robotic arm 110 with respect to the region of interest 140. The word "dynamically" here means that the localization unit is adapted to update the determined poses at a predefined frequency. For instance, the frequency of those updates is comprised between 5 Hz and 1500 Hz. Advantageously, this frequency is customizable depending on the kind of treatment to perform and/or depending on the progress of the treatment. The frequency can thus be modified during a single surgery, for instance between two successive cuts.

The first subsystem comprises positional sensors - not shown - adapted to determine, dynamically, a current geometric configuration of the passive tool holder 120. As detailed below, the positional sensors can for instance be realized as encoders. Based on this geometric configuration, and on registered geometries of the passive tool holder 120 and of the surgical tool 130, the control unit 102 is adapted to determine the relative pose of the surgical tool 130 with respect to the flange 112 of the robotic arm 110, and especially the relative pose of the active portion 133 of this surgical tool 130 with respect to the flange 112. As mentioned above, the pose of the planar area forming the working range R depends on the pose of the flange 112. Thus, the control unit can determine the relative pose of the active portion 133 of the surgical tool 130 with respect to the working range R based on the determined pose of the surgical tool 130 with respect to the flange 112 and on the stored pose of the flange with respect to the working range R.

According to the illustrated embodiment, the second subsystem 210 is an optical tracking unit, but it could be of any other known technology, such as an electromagnetic tracking unit, an inertial tracking unit or a combination of those, without departing from the scope if the invention. The second subsystem 210 comprises at least a first tracker 211 rigidly fixed to the robotic arm 110, a second tracker 212 rigidly attached to the anatomical structure to be treated and a detecting system, formed as a camera system 213 in the represented optical tracking unit, adapted to detect the current pose of the trackers 211, 212 and to determine, based on said current poses of the trackers and on a known geometry between each tracker and the corresponding tracked objects, the relative pose of the robotic arm 110, and especially of its flange 112, with respect to the region of interest 140 of the anatomical structure. As schematically illustrated, the first tracker 211 can for instance be attached to the last link 113 of the robotic arm 110, i.e., the link ending with the flange 112 to which the passive tool holder 120 is connected. Alternatively, the first tracker 211 could be arranged on the second to last link. According to another alternative, the first tracker 211 could be arranged on the base 101 of the computer-assisted surgery system 100 within the scope of the invention.

Optionally, the computer-assisted surgery system 100 comprises at least one display device 160 adapted to display a representation computed by the control unit 102 of the current poses of the robotic arm 110 and of the region of interest 140. Thus, the user of the system can appreciate, at any time the relative poses of said objects. Advantageously, the representation of the region of interest can be updated, by the control unit 102, as the surgical tool treats said region of interest. For instance, parts of the region of interest which have already been treated, i.e., parts where the surgical tool 130 has already been operated, can be represented with different colors or textures. This is achieved thanks to the indirect tracking of the surgical tool with respect to the region of interest. Indeed, the first subsystem of the localization unit permits to determine the current pose of the surgical tool with respect to the flange and to determine, consequently, the current pose of the surgical tool, and especially of its active portion, with respect to the working range. The second subsystem 210 is adapted to determine the current relative pose of the flange with respect to the region of interest. Thus, by combining information retrieve from the first subsystem and from the second subsystem, the localization unit is adapted to determine, dynamically, the pose of the surgical tool 130, and especially of its active portion 133, with respect to the region of interest 140.

According to the illustrated embodiment, the display device 160 is formed as a display arranged on arm attached to the robotic arm 110. Obviously, this is merely an example and the display could be positioned on another part of the surgical system, such as its base, within the scope of the invention. Alternatively, the display could also be attached to an operating table 141 where a patient lies. According to yet another alternative, the display device could be an augmented reality device positioned between the eyes of the user and the region of interest, such as augmented reality glasses, goggles, or panel.

As evoked above, the surgical system of the invention is adapted to help a user performing a cut of a planar region of interest. For instance, such system can thus be used for performing an osteotomy, or to prepare a bone to receive an artificial implant for instance in the case of a total or partial knee arthroplasty, or of a hip arthroplasty or an ankle arthroplasty etc....

The control unit 102 is adapted to receive from the localization unit 210, dynamically, the relative pose of the working range R with respect to the region of interest 140 and to dynamically compute and send at least one first instruction to at least one of the motorized joints in order for the working range to overlap, at least partially, the region of interest. By "dynamically", we here mean that the concerned pose is regularly updated. The frequency of this updating is comprised between 5 Hz and 1500 Hz. Advantageously, this frequency is customizable depending on the kind of treatment to perform and/or depending on the progress of the treatment. The frequency can thus be modified during a single surgery, for instance between two successive cuts.

Thus, the computer-assisted surgery system 100 of the invention ensures that its user only performs cuts within the planned plane, i.e. the planned region of interest. In other words, the computer-assisted surgery system of the invention is adapted to compensate the potential movements of the region of interest, which can be due to the patient respiration or to efforts generated by the treatment itself, especially when said treatment implies cutting bones. Optionally, the control unit can be adapted to filter the first instruction(s), for instance by not considering the movements of the patient which present a frequency above a predetermined threshold, this threshold being for instance set so as to not consider potential vibrations of the trackers while computing the first instruction(s).

To limit parasitic forces which could be inadvertently applied by the user on the passive tool holder, and which could result in an important deviation of the surgical tool 130 from the planar region of interest, the passive tool holder is advantageously designed to present a small offset with respect to the flange 112 of the robotic arm - figures are only here to illustrate the invention and or not drawn to scale. This results in a compact passive tool holder, but which presents a small working range, and especially, a working range which can be smaller than the planar region of interest. As more detailed below, the control unit 102 is adapted to compute at least one second instruction(s) adapted to be sent to at least one of the motorized joints of the robotic arm in order to displace the flange 112 of the robotic arm so as for the active portion of the surgical tool to be located at the targeted position within the working range R regardless the pose of said working range. According to the invention, this targeted position is defined as a particular point of the working range which can be characterized by its position relative to the border of the working range. As mentioned above, this relative position of the targeted position with respect to the border of the working range is registered in the storage unit. For instance, this targeted position can be a center of the working range, i.e. a point of the working range equidistant from all the points forming its border.

As previously mentioned, the pose of the working range depends on the pose of the flange. By displacing the flange 112, the computer-assisted surgery system of the invention therefore permits the robotic arm to follow the displacements of the surgical tool, thus ensuring that the user of the system is able to treat the entirety of the planar region of interest. In the present document, the words "updated working range" are used to designate a new pose of the working range, such modification of its pose being due to a displacement of the flange.

Optionally, the computer-assisted surgery system 100 of the invention can also comprise at least one force and/or torque sensor - not illustrated - arranged between the flange of the robotic arm and the power tool of the surgical tool. Advantageously, this force and/or torque sensor is arranged between the flange of the robotic arm and the passive tool holder. This force and/or torque sensor is adapted to monitor forces and/or torques applied on the passive arm and to compare the measured values to different thresholds.

According to an aspect of the invention, if the value measured by the force and/or torque sensor is greater than a first threshold, the control unit can be adapted to alert the user of the system, for instance thanks to sensorial feedback, such as visual feedback by turning a light on, auditive feedback by playing a sound or tactile feedback for instance. At least a second threshold, greater than the first threshold, can also be set and the control unit be adapted to stop the computer-assisted surgery system if the measured value is above said second threshold, as a safety measure. Obviously, intermediate threshold(s) with adapted feedback(s) can also be implemented within the scope of the invention.

Optionally, the storage unit can store deformation model(s) of the robotic arm and/or of the passive tool holder. The value measured by the force and/or torque sensor can, in this particular case, be used concurrently with the stored deformation model(s) when computing the first instruction(s). The system thus permits to compensate the measured deformations and ensures that the surgical tool remains within the plane in which extends the planar region of interest.

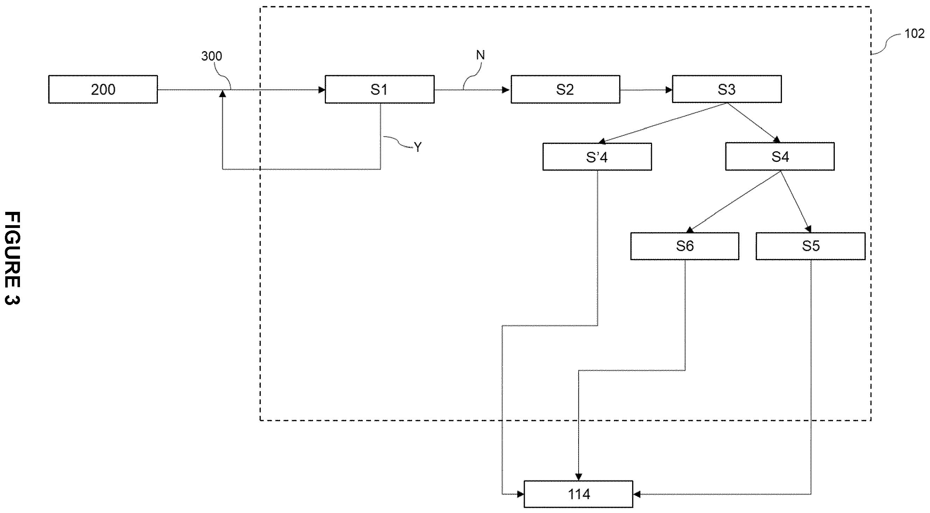

Figure 3 schematically illustrates the computing and the execution of the second instruction(s).

The control unit 102 first receives, from the localization unit 200, an information 300 related to the current pose of the surgical tool with respect to the working range. The control unit 102 is then adapted, during a first step S1, to determine a relative position of the active portion of the surgical tool with respect to the working range, based on the received information 300 and on the stored geometry of the surgical tool, and to compare this determined relative position of the active portion of the surgical tool with respect to the working range, with the targeted position stored in the storage unit.

If it is determined that the active portion of the surgical tool is located at the targeted position (Y), the control unit 102 keeps monitoring the evolution of the pose of the surgical tool with respect to the working range.

If it is determined that the active portion of the surgical tool is not located at the targeted position (N), the control unit 102 is adapted, in a second step S2, to determine a pose of an updated working range which would permit the surgical tool, and especially its active portion, to be located at the targeted position within such updated working range. In a third step S3, the control unit 102 determine an updated pose of the flange permitting the working range to reach its updated pose.

The control unit 102 can then, in a fourth step S4, determine if the updated working range will extend beyond the protective boundary of the region of interest or if such updated working range will remain within said protective boundary.

If it is determined that the updated working range will remain within the protective boundary, the control unit 102 is adapted to, in a fifth step S5, compute at least one second instruction adapted to be sent to at least one of the motorized joints in order to displace the flange of the robotic arm to its updated pose. The control unit 102 can finally send the second instruction(s) to the concerned motorized joint(s) 114 and execute such second instruction(s).

If it is determined that the updated working range will extend beyond the protective boundary, the control unit 102 is adapted to, in a sixth step S6 modify the updated pose of the working range in order for a border of the modified updated working range to match the protective boundary of the region of interest, modify the updated pose of the flange so as for the working range to be displaced to the modified updated working range and compute at least one second instruction(s) adapted to be sent to at least one of the motorized joints 114 of the robotic arm in order to displace the flange of the robotic arm to its modified updated pose. The control unit can then send the at least one second instruction(s) to the concerned motorized joint(s) 114 and execute such second instruction(s).

Advantageously, the present invention thus permits to protect the tissues surrounding the region of interest by physically making it impossible, or at least harder, for the surgical tool, and especially its active portion, to reach said surrounding tissues.

Alternatively, the control unit 102 can be adapted to compute, send and execute the second instruction(s) directly, without implementing the fourth, fifth or sixth steps. According to this alternative, the at least one second instruction(s) can thus be computed during the third step S3, concurrently to the determining of the updated pose of the flange permitting the working range to reach its updated pose, or in a distinct step S'4 within the scope of the present invention. According to this alternative, the user of the system is the only one to estimate when to stop the surgical tool, for instance based on his/her sensation as he/she performs the treatment of the region of interest. Indeed, as the passive tool holder is deprived of motors, the user has direct feedback of his/her work.

All those steps are described as being performed successively, but they could be realized simultaneously, or with a different order than the one described, at least for part of them, within the scope of the invention.

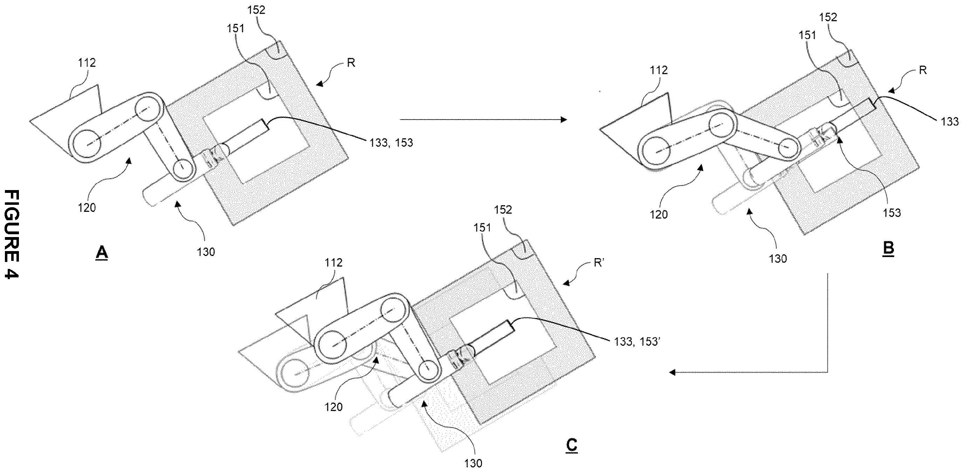

According to a particular configuration of the invention, as for instance illustrated on on figure 4, the control unit 102 can also be adapted to execute the second instruction(s) only when the active portion 133 of the surgical tool 130 enters the peripheral zone 152 of the working range R. The control unit 102 is thus adapted to determine, based on the information 300 received from the localization unit 200, if the active portion 133 of the surgical tool 130 is within the central zone 151 or the peripheral zone 152 of the working range R and to send and execute the second instruction(s) only if said active portion 133 is located within the peripheral zone 152.

Figure 4 especially illustrate a first situation A, wherein the active portion 133 of the surgical tool 130 is at the targeted position 153 of the working range R, a second situation B wherein the surgical tool 130 has been displaced, while performing the treatment, the active portion 133 of the surgical tool 130 thus not being at the targeted position 153 anymore, and a third situation C, wherein the control 102 has computed, sent and executed the at least one second instruction(s), resulting in a displacement of the flange 112, itself resulting in a displacement of the working range to the pose of the updated working range R', and thus permitting the active portion 133 of the surgical tool 130 to be located at the targeted position 153' of the updated working range R'. As schematically illustrated on the second situation B, the control unit is adapted to execute the at least one second instruction(s), when the surgical tool 130 reaches the peripheral zone 152 of the working range R.

While a general configuration of the invention ensures that the user manipulates an ergonomic passive tool holder at any time, the particular configuration reduces the frequency of the displacements of the robotic arm which can be disturbing for some users. The computer-assisted surgery system of the invention can thus be operated according to either of those configurations, depending on the preferences of the user, or on the kind of surgery to be realized for instance. Obviously, other factors can be considered to choose between those two configurations. Also, some steps of a surgery can be performed with the system in one configuration and other steps of the same surgery can be performed with the system in its other configuration.

Advantageously, the control unit can be adapted to select only some of the displacements of the surgical tool to be considered for determining the updated pose of the working range, based on at least one parameter related to the displacement of the surgical tool. For instance, this at least one parameter can be one or several of the following: an acceleration of the displacement, a displacement speed, a frequency of this displacement or an amplitude of said displacement. It is understood that those parameters are determined based on several successive poses received by the control unit which can then gather those poses in order to determine at least one parameter related to the displacement of the surgical tool. Obviously other parameters could be used to realize the selection.

The control unit is for instance configured to compare the at least one parameter with a predefined range and to ignore the displacement(s) having a parameter outside said predefined range when determining the updated pose of the working range. For instance, if the surgical tool is an oscillating saw, this selection, whether based on the frequency, on the speed, on the amplitude or on the acceleration, avoids oscillations of the robotic arm, while still ensuring that the robotic arm follows a more general direction of displacement of the oscillating saw.

According to an aspect of the invention, the control unit can for instance be adapted to ignore the displacements of the surgical tool which present a frequency greater than a predefined threshold while determining the updated pose of the working range. This threshold can for instance be set at 1Hz. Advantageously, this threshold is set at 0,5Hz. According to another aspect of the invention, the control unit can be adapted to ignore the displacements of the surgical tool which present an acceleration greater than a predefined threshold determining the updated pose of the working range.

Referring to figure 5a to 7, we are now going to describe three different embodiments of the present invention. Those embodiments differ from one another in the geometry of their respective passive tool holder 120. Thus, only the different passive tool holders 120 are represented on figures 5a to 7, the description given above concerning the rest of the system applying *mutatis mutandis* to each of those embodiments.

Figures 5a and 5b illustrate perspective views of the passive tool holder 120 according to two variants of the first embodiment illustrated on figures 1 and 2.

According to the first embodiment of the invention, the passive tool holder 120 comprises at least three axes A1, A2, A3 forming three distinct passive joints 121. The words "passive joints" are here used as opposed to "motorized joints" as they are manually displaceable and deprived of motors. Two adjacent passive joints 121 define one segment 123 of the passive arm 120. Consequently, the passive arm 120 comprises at least two segments 123 linked to one another thanks to a first passive joint 121a. A second passive joint 121b is formed between the passive tool holder 120 and the flange 112 of the robotic arm and a third passive joint 121c is formed between the passive tool holder 120 and the surgical tool 130. The first axis A1 associated with the first passive joint 121a, the second axis A2 associated with the second passive joint 121b and the third axis A3 associated with the third passive joint 121c are parallel to one another. Those axes A1, A2, A3 are also perpendicular to a plane including the working range of the surgical tool 130. The surgical tool 130 can thus be displaced within a plane thanks to the first and second passive joints 121a, 121b, and it can be rotated around the third axis A3 forming the third passive joint.

The passive tool holder 120 further comprises at least three positional sensors respectively embedded within each passive joint 121. According to an aspect of the invention, each passive joint 121 is equipped with one of the positional sensors. The positional sensors are adapted to determine, dynamically, an angular position of the passive joint 121 to which it is associated, and to send a corresponding information to the control unit 102. Those positional sensors thus form part of the first subsystem of the localization unit. For instance, those positional sensors can be angular sensors, such as encoders. For instance, the encoders can be absolute encoders. If so, the storage unit also stores an association of the values of each encoder with specific geometries of the passive arm. Thus, the control unit can determine, dynamically, the current geometry of the passive tool-holder by using the values of the encoders. Optionally, at least one brake can be embedded in at least one of the passive joints 121. For instance, this brake can be an electromagnetic brake.

According to the variant of this first embodiment illustrated on figure 5b, the passive tool holder 120 comprises at least one mechanical stop 122 limiting the possible movements of the passive tool holder and, consequently, the working range of the surgical tool 130. Advantageously, using those mechanical stops 122 prevents the user to come too close to the singularities of the system, i.e. to close to positions in space the system cannot reach. Indeed, if a singularity of the system is reached, or nearly reached to be specific, the computer-assisted surgery system stops and needs to be restarted. Obviously, such a situation must be avoided during any surgery.

According to the illustrated embodiment, the mechanical stops 122 are formed as a single piece with the links of the passive tool holder. Optionally, those mechanical stops 122 could be removably mounted on the passive tool holder. For instance, the mechanical stops 122 can be mounted, respectively, on an elastic return device, such as a spring, so as to be removable. Advantageously, this particular feature permit to invert the first passive joint 121a if needed.

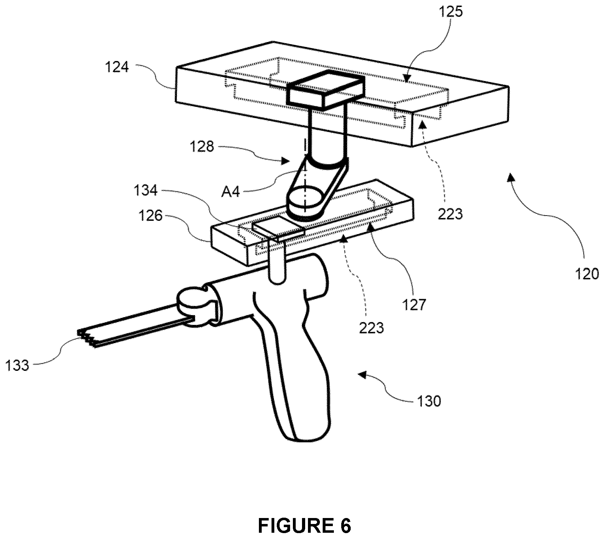

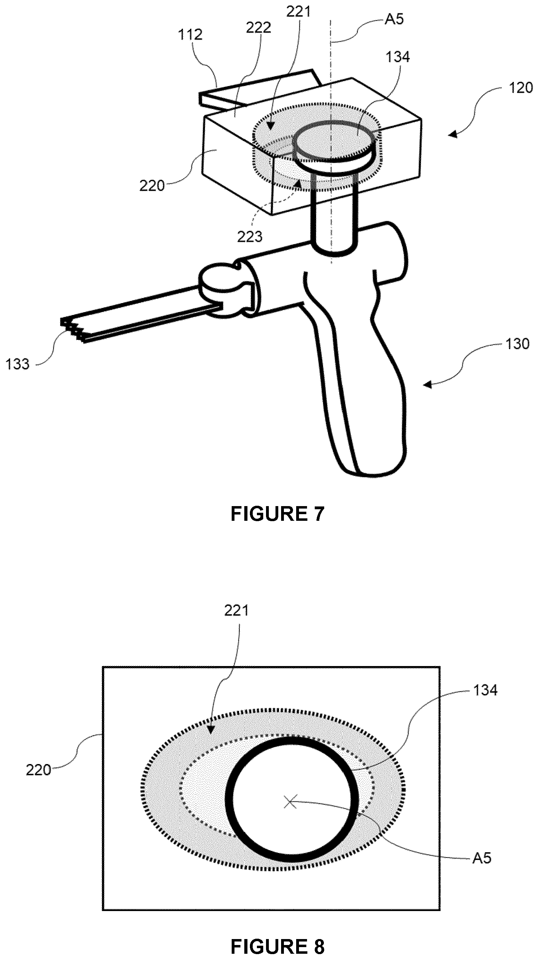

Figures 6 and 7 illustrate a second and a third embodiments of the invention wherein the passive tool holder 120 is formed as at least one opened housing which comprises at least one guiding device adapted to guide and constraint the displacements of the surgical tool 130 within its working range. The opened housing comprises at least one opening 223 adapted to cooperate with an attaching part 134 of the surgical tool 130.

According to the second embodiment illustrated on figure 6, the passive tool holder 120 comprises a first opened housing 124 equipped with at least one first rail 125 and a second opened housing 126 equipped with at least one second rail 127. The passive tool holder 120 further comprises an L-shaped segment 128, a first end of the L-shaped segment 128 being received in the first opened housing 124 and guided by the first rail 125 and a second end of this L-shaped segment 128 being received in the second opened housing 126 and guided by the second rail 127. It is understood that both those ends are adapted to be freely displaced along their respective rails 125, 127, thus allowing displacements of the surgical tool 130 within a plane. The second end of the L-shaped segment 128 also forms a rotatable joint allowing the surgical tool 130 to rotate around an axis A4 perpendicular to the plane. As for the first embodiment, the passive tool holder 120 according to the second embodiment encompasses positional sensors - not shown - to determine a current configuration of the passive tool holder 120. For instance, positional sensors according to the second embodiment of the invention can comprise two linear sensors, such as potentiometers, respectively arranged to detect and measure displacements applied along the first rail 125 and the second rail 127, and one angular sensor-also called rotational sensor or encoder-adapted to detect and measure the rotations applied on the surgical tool 130. Optionally, some degrees of freedom of the passive tool holder can be locked. For instance, the displacement of the first end of the L-shaped segment 128 along the first rail 125 can be locked, as the rotation around the axis A4, while the displacements of the second end of the L-shaped segment 128 along the second rail 127 remains free. Such a configuration can for instance be useful if the surgical tool is a drill adapted to form a hole in a bone. Any other combination is possible as long as at least one degree of freedom of the passive tool holder 120 remains free.

Figure 7 illustrates the third embodiment of the invention. As mentioned above, the passive tool-holder according to this third embodiment comprises an opened housing 220 equipped with a guiding device. According to this third embodiment, the guiding device is formed as a blind hole 221 arranged in a wall 222 of the opened housing 220 which faces the opening 223 of said opened housing 220. According to the illustrated embodiment, this blind hole 221 can for instance present a circular shape. Alternatively, as for instance illustrated on figure 8 which is a partial top view of a variant of the third embodiment, this blind hole 221 can be realized according to different shapes, such as an oblong shape. Alternatively, this blind hole 221 could present a rectangular shape, a triangular shape, a square shape or any other shape within the scope of the invention. It is understood that the shape of this blind hole 221 participates to determine the geometry of the working range of the surgical tool.

As shown, the blind hole 221 is adapted to receive an attaching part 134 of the surgical tool 133. Advantageously, and as illustrated, the attaching part 134 of the surgical tool 130 presents a shape complementary to the shape of the blind hole 221. It is understood that the attaching part 134 is adapted to be displaced freely within said blind hole 221 so that the surgical tool 130 can be freely moved along at least three degrees of freedom upon which at least two translations - defining partially the working range - and one rotation, around an axis A5 perpendicular to the plane wherein the attaching part 134 can be displaced. For instance, the relative displacement of the attaching part 134 with respect to the blind hole 221 are realized through a ball bearing device - not illustrated. Alternatively, they can be equipped with polytetrafluoroethylene dots adapted to slide with respect to each other.

As well as for the two other embodiments, the passive tool holder 120 according to the third embodiment comprises at least one positional sensor adapted to determine a current position of an attaching part of the surgical tool thanks to which such surgical tool is attached to the passive tool holder. This sensor can for instance be non-contact sensor, such as a magnetic or optical sensor.

## Claims

1. Computer-assisted surgery system (100) for treating a planar region of interest (140) of an anatomical structure with a surgical tool (130), the computer-assisted surgery system (100) comprising:
• a robotic arm (110) with at least three motorized joints (114),
• a passive tool holder (120) attached to a flange (112) of the robotic arm (110),
• the surgical tool (130) attached to the passive tool holder (120), the surgical tool (130) being adapted to be displaced, manually, within a working range (R), the working range (R) being formed as a planar area, a geometry of this working range (R) being determined by a geometry of the passive tool holder (120) and a pose of this working range (R) being determined by a pose of the flange (112),
• a storage unit (103) storing, at least, a geometry of the surgical tool (130) and a targeted position (153) of an active portion (133) of the surgical tool (130) within the working range (R),
• a localization unit adapted to determine, dynamically, a relative pose of the working range (R) with respect to the region of interest (140),
• a control unit (102) adapted to receive from the localization unit the relative pose of the working range (R) with respect to the region of interest (140) and to dynamically compute and send at least one first instruction to at least one of the motorized joints (114) in order for the working range (R) to overlap, at least partially, the region of interest (140),
the control unit (102) being configured to:
• receive from the localization unit, dynamically, a relative position of the surgical tool (130) with respect to the working range (R),
• determine a relative position of an active portion (133) of the surgical tool (130) with respect to the working range (R) based on the received relative position of the surgical tool (130) with respect to the working range (R) and on the stored geometry of the surgical tool (130),
• compare the determined relative position of the active portion (133) of the surgical tool (130) with respect to the working range (R) with the stored targeted position (153) of the active portion (133) of the surgical tool (130) within the working range (R),
• determine a pose of an updated working range (R') so as for the active portion (133) of the surgical tool (130) to be located at the targeted position (153') within said updated working range (R'), if the received relative position of the active portion (133) of the surgical tool (130) with respect to the working range (R) is different from the targeted position (153),
• determine an updated pose of the flange (112) so as for the working range (R) to match the determined pose of the updated working range (R').

2. Computer-assisted surgery system (100) according to the preceding claim, wherein the region of interest (140) is defined by a contour, a first part of this contourforming a protective boundary and a second part of this contour forming an entry zone to the region of interest (140), and wherein the control unit (102) is further configured to
• determine if the updated working range (R') will remain within the protective boundary of the region of interest (140),
• compute at least one second instruction(s) adapted to be sent to at least one of the motorized joints (114) of the robotic arm (110) in order to displace the flange (112) of the robotic arm (110) to its updated pose, if it is determined that the updated working range (R') will remain within the protective boundary of the region of interest (140),
• execute the at least one second instruction(s).

3. Computer-assisted surgery system (100) according to any of the preceding claims, wherein the region of interest (140) is defined by a contour, a first part of this contour forming a protective boundary and a second part of this contour forming an entry zone to the region of interest (140), and wherein the control unit (102) is further configured to
• determine if the updated working range (R') will extend beyond the protective boundary of the region of interest (140),
• modify the updated pose of the working range (R) in order for a border (150) of the modified updated working range (R') to match the protective boundary of the region of interest (140), if it is determined that the updated working range (R') will extend beyond the protective boundary of the region of interest (140),
• modify the updated pose of the flange (112) so as for the working range (R) to be displaced to the modified updated working range (R'),
• compute at least one second instruction(s) adapted to be sent to at least one of the motorized joints (114) of the robotic arm (110) in order to displace the flange (112) of the robotic arm (110) to its modified updated pose,
• execute the at least one second instruction(s).

4. Computer-assisted surgery system (100) according to any of claims 2 or 3, wherein the working range (R) comprises a central zone (151) surrounded by a peripheral zone (152), and wherein the control unit (102) is configured to
• execute the second instruction(s) as soon as the surgical tool (130) enters the peripheral zone (152).

5. Computer-assisted surgery system (100) according to the preceding claim, wherein the peripheral zone (152) represents up to 30% of the working range (R).

6. Computer-assisted surgery system (100) according to any of the preceding claims, wherein determining the pose of the updated working range (R') comprises:
• determining at least one parameter related to the displacement of the surgical tool (130) based on the received relative positions of the surgical tool (130) with respect to the working range (R);
• selecting the displacements to be considered for determining the pose of the updated working range (R'), based on the at least one parameter.

7. Computer-assisted surgery system (100) according to the preceding claim, wherein the at least one parameter is one or several of the following:
• a displacement acceleration of the surgical tool (130),
• a displacement speed of the surgical tool (130),
• a displacement amplitude of the surgical tool (130),
• a frequency of the displacement of the surgical tool (130).

8. Computer-assisted surgery system (100) according to any of the claims 6 or 7, wherein selecting the displacements to be considered for determining the pose of the updated working range (R') comprises:
• determining the at least one parameter related to the displacement of the surgical tool (130),
• comparing the determined parameter(s) with a predefined range,
• ignoring the displacements having parameter(s) outside the predefined range while determining the pose of the updated working range (R').

9. Computer-assisted surgery system (100) according to any of the preceding claims, wherein the localization unit comprises:
• a first subsystem adapted to determine, dynamically, the relative poses of the surgical tool (130) with respect to the working range (R),
• a second subsystem adapted to determine, dynamically, the relative poses of the flange (112) of the robotic arm (110) with respect to the region of interest (140),
the first subsystem comprising at least one positional sensor adapted to determine, dynamically, a geometry of the passive tool holder (120), and the second subsystem comprising an optical tracking unit and/or an electromagnetic tracking unit and/or an inertial tracking unit adapted to determine, dynamically, the relative pose of the flange (112) with respect to the anatomical structure.

10. Computer-assisted surgery system (100) according to any of the preceding claims, wherein the passive tool holder (120) comprises at least a first segment (123) and a second segment (123) linked to one another thanks to a first passive joint (121a) rotatable around a first axis (A1), wherein the first segment (123) is attached to the flange (112) of the robotic arm (110) thanks to a second passive joint (121b) rotatable around a second axis (A2) parallel to the first axis (A1), and wherein the second segment (123) is attached to an attaching part (134) of the surgical tool (130) thanks to a third passive joint (121c) rotatable around a third axis (A3) parallel to the first axis (A1) and to the second axis (A2).

11. Computer-assisted surgery system (100) according to the preceding claim, wherein each passive joint (121a, 121b, 121c) is equipped with at least one angular sensor adapted to determine, dynamically, an angular position of the concerned passive joint (121a, 121b, 121c), the at least one angular sensor being part of the first subsystem of the localization unit.

12. Computer-assisted surgery system (100) according to any of claims 1 to 9, wherein the passive tool holder (120) comprises at least one opened housing (124, 126, 220) which cooperates with an attaching part (134) of the surgical tool (130), the at least one opened housing (124, 126, 220) comprising a guiding device (125, 127, 221) adapted to guide and limit the displacement of the surgical tool (130) within the working range (R) and wherein the attaching part (134) of the surgical tool (130) allows rotation of the surgical tool (130) around an axis perpendicular to the working range (R).

13. Computer-assisted surgery system (100) according to the preceding claim, wherein the passive tool holder (120) comprises a first opened housing (124) and a second opened housing (126), the guiding device comprising at least a first rail (125) mounted within the first opened housing (124) and a second rail (126) mounted within the second opened housing (126).

14. Computer-assisted surgery system (100) according to claim 12, wherein the guiding device is formed as a blind hole (221) formed within the opened housing (124, 126, 220).

15. Computer-assisted surgery system (100) according to any of the preceding claims, comprising at least one force or torque sensor arranged between the flange (112) of the robotic arm (110) and an attaching part (134) of the surgical tool (130).
